# EUROPÄISCHE PATENTANMELDUNG

(11) **EP 4 851 132 A1**
(43) Veröffentlichungstag der Anmeldung: **09.12.2026**
(21) Anmeldenummer: 25181047.9
(22) Anmeldetag: 05.06.2025
(51) Int. Cl.: C07C 67/00, C07C 69/24

(54) **VERFAHREN ZUR ALKOXYCARBONYLIERUNG VON ETHYLENISCH UNGESÄTTIGTEN VERBINDUNGEN UNTER EINSATZ VON CO2 UND EINES BIMETALLISCHEN KATALYSATORSYSTEMS**

(71) Anmelder: Evonik Oxeno GmbH & Co. KG, 45772 Marl (DE)
(72) Erfinder: FRANKE, Robert, 45772 Marl (DE); BELLER, Matthias, 18211 Ostseebad Nienhagen (DE); JACKSTELL, Ralf, 18106 Rostock (DE); HUANG, Weiheng, Qingyuan, Guangdong (CN); TIAN, Xinxin, 030000 Taiyuan, Shanxi Provinz (CN); JIAO, Haijun, 18055 Rostock (DE)
(74) Vertreter: Evonik Patent Association

(57) **Zusammenfassung**

Verfahren zur Alkoxycarbonylierung von ethylenisch ungesättigten Verbindungen unter Einsatz von CO₂ und eines bimetallischen Katalysatorsystems.

## Beschreibung

Die Erfindung betrifft ein Verfahren zur Alkoxycarbonylierung von ethylenisch ungesättigten Verbindungen unter Einsatz von CO₂ und eines bimetallischen Katalysatorsystems.

In EP 4 001 256 A1 wird ein Verfahren zur Alkoxycarbonylierung von ethylenisch ungesättigten Verbindungen unter Einsatz von benzolbasierten Diphosphinliganden und Aluminiumtriflat beschrieben.

Die technische Aufgabe der Erfindung ist die Bereitstellung eines neuen Verfahrens, welches eine gesteigerte Ausbeute an Ester liefern soll.

Die Aufgabe wird gelöst durch ein Verfahren nach Anspruch 1.

Verfahren umfassend die Verfahrensschritte:
a) Vorlegen einer ethylenisch ungesättigten Verbindung;
b) Zugabe eines Liganden gemäß Formel (I): 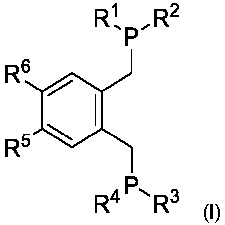 wobei
   R¹, R², R³, R⁴ jeweils für -(C₁-C₆)-Alkyl stehen,
   R⁵, R⁶ jeweils für einen Rest stehen ausgewählt aus: -H, -O-(C₁-C₆)-Alkyl, -(C₁-C₆)-Alkyl;
c) Zugabe einer Verbindung, welche Pd umfasst;
d) Zugabe einer Verbindung, welche Ir umfasst;
e) Zugabe einer Zinkverbindung welche ausgewählt ist aus:
   Zn(SO₃C₆H₄CH₃)·xH₂O, Zn(SO₃CH₃)·xH₂O, Zn(OTf)₂, Zn(SO₄)₂;
f) Zugabe eines Alkohols;
g) Zuführen von CO₂ und H₂;
h) Erwärmen des Reaktionsgemisches aus a) bis g), wobei die ethylenisch ungesättigte Verbindung zu einem Ester umgesetzt wird.

In einer Variante des Verfahrens stehen R¹, R², R³, R⁴ für -*^{t}*Bu.

In einer Variante des Verfahrens sind R⁵, R⁶ ausgewählt aus: -H, -O-CH₃.

In einer Variante des Verfahrens weist der Ligand die Struktur (1) oder (2) auf:

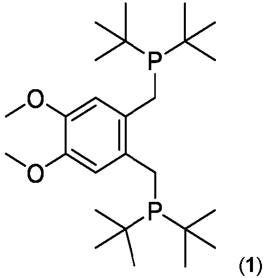

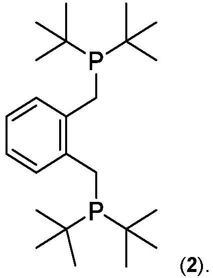

In einer Variante des Verfahrens weist der Ligand die Struktur (1) auf:

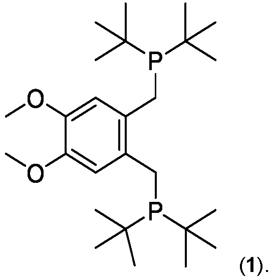

In einer Variante des Verfahrens weist der Ligand die Struktur (2) auf:

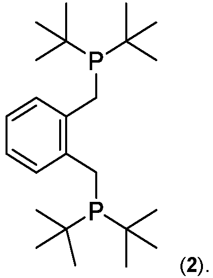

In einer Variante des Verfahrens ist die Verbindung in Verfahrensschritt c), welche Pd umfasst, ausgewählt aus: Palladiumdichlorid, Palladium(II)-acetylacetonat, Palladium(II)-acetat (Pd(OAc)₂), Dichloro(1,5-cyclooctadiene)palladium(II), Bis(dibenzylideneaceton)palladium (Pd(dba)₂), Bis(acetonitrile)dichloropalladium(II), Palladium(cinnamyl)dichlorid, Palladium(II)bis(rifluormethansulfonat) (Pd(TFA)₂.

In einer Variante des Verfahrens ist die Verbindung in Verfahrensschritt c), welche Pd umfasst, Pd(dba)₂.

In einer Variante des Verfahrens ist die Verbindung in Verfahrensschritt d), welche Ir umfasst, Ir(acac)(CO)₂.

In einer Variante des Verfahrens ist die Zinkverbindung ausgewählt aus:
Zn(SO₃C₆H₄CH₃)·xH₂O, Zn(SO₃CH₃)·xH₂O, Zn(OTf)₂.

In einer Variante des Verfahrens ist die Zinkverbindung Zn(OTf)₂.

In einer Variante des Verfahrens ist der Alkohol in Verfahrensschritt f) ausgewählt aus: Methanol, Ethanol, 1-Propanol, 1-Butanol, 1-Pentanol, 1-Hexanol, 2-Propanol, tert-Butanol, 3-Pentanol, Cyclohexanol, Phenol.

In einer Variante des Verfahrens ist der Alkohol in Verfahrensschritt f) Methanol.

In einer Variante des Verfahrens erfolgt das Zuführen von CO₂ mit einem Druck im Bereich von 1 MPa (10 bar) bis 5 MPa (50 bar).

In einer Variante des Verfahrens erfolgt das Zuführen von H₂ mit einem Druck im Bereich von 1 MPa (10 bar) bis 5 MPa (50 bar).

In einer Variante des Verfahrens erfolgt das Erwärmen auf eine Temperatur im Bereich von 80 °C bis 160 °C.

Im Folgenden soll die Erfindung anhand von Ausführungsbeispielen näher erläutert werden.

### Versuchsbeschreibung allgemein

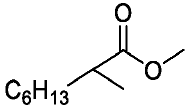

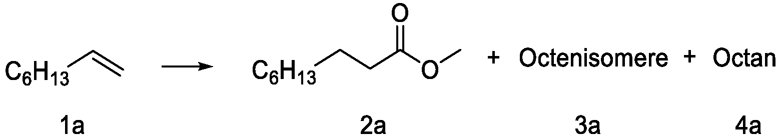

Die festen Ausgangsstoffe Palladiumvorstufe Pd(dba)₂, Co-Katalysator, Ligand und Additiv wurden abgewogen und im Autoklav in ein 12-ml-Schraubdeckel-Vial mit 6-mm-Magnetrührstab gegeben. Das Vial wurde mit einem Teflon/Silikon-Septum und einem Phenolharzdeckel verschlossen und dann aus dem Autoklav ausgetragen. Das Vial wurde mit einer Nadel unter Argonstrom an eine Schlenklinie angeschlossen. Mit einer Spritze wurden destilliertes Methanol (2,0 ml) und 1-Octen (157 µl, 1,0 mmol) eingespritzt. Das Vial wurde auf einer Legierungsplatte gehalten und in einen Autoklaven (300 ml) der Reihe 4560 von Parr gegeben. Der Autoklav wurde bei Raumtemperatur zweimal mit Stickstoffgas gespült. Außerdem wurde zweimal mit Kohlendioxid gespült und Kohlendioxid bis 20 bar aufgepresst, wonach Wasserstoff (mit 20 bar) bis zu einem Gesamtdruck von 40 bar zugeführt wurde. Der Ansatz wurde auf 140 °C erhitzt und 20 Stunden gerührt (600 U/min). Danach wurde der Autoklav mit Eiswasser abgekühlt, wonach das Gas vorsichtig abgelassen wurde. Die Lösung wurde mit Isooctan (83 µl, 0,5 mmol) als internem Standard versetzt, über Celite filtriert und durch Gaschromatographie analysiert.

### Variation des Co-Katalysators

### Reaktionsbedingungen:

**1a** (1,0 mmol), Pd(dpa)₂ (0,5 mol%), Co-Katalysator (0,05 mol%), d^{t}bpx (**2**) (1,0 mol%), Zn(OTf)₂ (5 mol%), MeOH, CO₂ (20 bar), H₂ (20 bar), 140 °C, 20 h

**Tabelle 1**

| Co-Katalysator | Ausbeute **2a** [%] |
|---|---|
| - | < 1 |
| Fe₃(CO)₁₂ | < 1 |
| Ru₃(CO)₁₂ | 8 |
| Co₂(CO)₈ | 12 |
| Ir(acac)(CO)₂* | 25 |

| | |
|---|---|
| * erfindungsgemäßes Ausführungsbeispiel | |

### Variation des Liganden

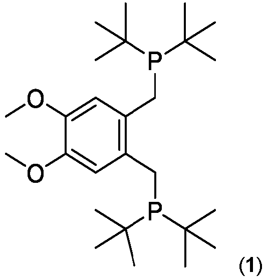

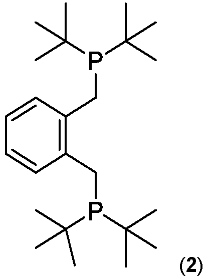

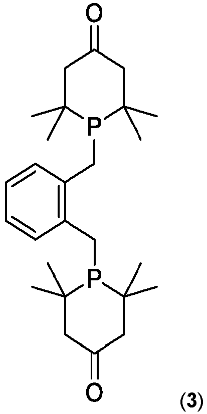

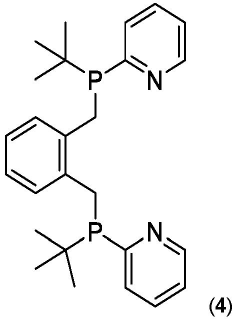

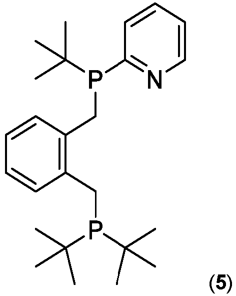

### Reaktionsbedingungen:

**1a** (1,0 mmol), Pd(dpa)₂ (0,5 mol%), Ir(acac)(CO)₂ (0,05 mol%), Ligand (1,0 mol%), Zn(OTf)₂ (5 mol%), MeOH, CO₂ (20 bar), H₂ (20 bar), 140 °C, 20 h

**Tabelle 2**

| Ligand | Ausbeute **2a** [%] |
|---|---|
| **(1)*** | 33 |
| **(2)*** | 25 |
| **(3)** | 8 |
| **(4)** | < 1 |
| **(5)** | 2 |

| | |
|---|---|
| * erfindungsgemäßes Ausführungsbeispiel | |

### Variation des Additivs

### Reaktionsbedingungen:

**1a** (1,0 mmol), Pd(dpa)₂ (0,5 mol%), Ir(acac)(CO)₂ (0,05 mol%), d*^{t}*bpx (**2**) (1,0 mol%), Additiv (5 mol%), MeOH, CO₂ (20 bar), H₂ (20 bar), 140 °C, 20 h

**Tabelle 3**

| Additiv | Ausbeute **2a** [%] |
|---|---|
| PTSA·H₂O | - |
| Al(OTf)₃ | - |
| Fe(OTf)₃ | - |
| ZnCl₂ | - |
| Zn(OAc)₂ | - |
| Zn(SO₃C₆H₄CH₃)·xH₂O* | 14 |
| Zn(SO₃CH₃)·xH₂O* | 23 |
| Zn(OTf)₂* | 25 |

| | |
|---|---|
| * erfindungsgemäßes Ausführungsbeispiel | |

Wie die durchgeführten Versuche zeigen, wird die gestellte Aufgabe durch ein erfindungsgemäßes Verfahren gelöst.

## Patentansprüche

1. Verfahren umfassend die Verfahrensschritte:
a) Vorlegen einer ethylenisch ungesättigten Verbindung;
b) Zugabe eines Liganden gemäß Formel (I): 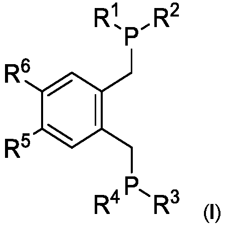 wobei
R¹, R², R³, R⁴ jeweils für -(C₁-C₆)-Alkyl stehen,
R⁵, R⁶ jeweils für einen Rest stehen ausgewählt aus: -H, -O-(C₁-C₆)-Alkyl, -(C₁-C₆)-Alkyl;
c) Zugabe einer Verbindung, welche Pd umfasst;
d) Zugabe einer Verbindung, welche Ir umfasst;
e) Zugabe einer Zinkverbindung welche ausgewählt ist aus:
Zn(SO₃C₆H₄CH₃)·xH₂O, Zn(SO₃CH₃)·xH₂O, Zn(OTf)₂, Zn(SO₄)₂;
f) Zugabe eines Alkohols;
g) Zuführen von CO₂ und H₂;
h) Erwärmen des Reaktionsgemisches aus a) bis g), wobei die ethylenisch ungesättigte Verbindung zu einem Ester umgesetzt wird.

2. Verfahren nach Anspruch 1,
wobei R¹, R², R³, R⁴ für -*^{t}*Bu stehen.

3. Verfahren nach einem der Ansprüche 1 bis 2,
wobei R⁵, R⁶ ausgewählt sind aus: -H, -O-CH₃.

4. Verfahren nach einem der Ansprüche 1 bis 3,
wobei der Ligand die Struktur (1) oder (2) aufweist:

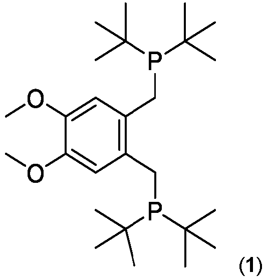

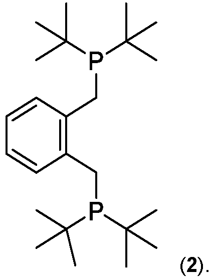

5. Verfahren nach einem der Ansprüche 1 bis **4,** wobei der Ligand die Struktur **(1)** aufweist:

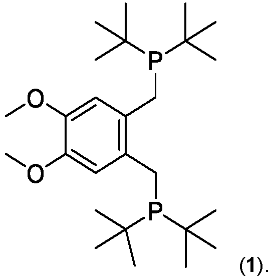

6. Verfahren nach einem der Ansprüche **1** bis **4,** wobei der Ligand die Struktur **(2)** aufweist:

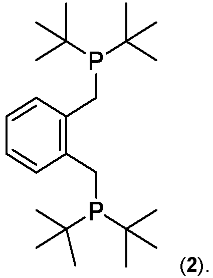

7. Verfahren nach einem der Ansprüche 1 bis 6,
wobei die Verbindung in Verfahrensschritt c), welche Pd umfasst, ausgewählt ist aus:
Palladiumdichlorid, Palladium(II)-acetylacetonat, Palladium(II)-acetat (Pd(OAc)₂), Dichloro(1,5-cyclooctadiene)palladium(II), Bis(dibenzylideneaceton)palladium (Pd(dba)₂), Bis(acetonitrile)dichloropalladium(II), Palladium(cinnamyl)dichlorid,
Palladium(II)bis(rifluormethansulfonat) (Pd(TFA)₂.

8. Verfahren nach einem der Ansprüche 1 bis 7,
wobei die Verbindung in Verfahrensschritt c), welche Pd umfasst, Pd(dba)₂ ist.

9. Verfahren nach einem der Ansprüche 1 bis 8,
wobei die Verbindung in Verfahrensschritt d), welche Ir umfasst, Ir(acac)(CO)₂ ist.

10. Verfahren nach einem der Ansprüche 1 bis 9,
wobei die Zinkverbindung Zn(OTf)₂ ist.

11. Verfahren nach einem der Ansprüche 1 bis 10,
wobei der Alkohol in Verfahrensschritt f) ausgewählt ist aus: Methanol, Ethanol, 1-Propanol, 1-Butanol, 1-Pentanol, 1-Hexanol, 2-Propanol, *tert*-Butanol, 3-Pentanol, Cyclohexanol, Phenol.

12. Verfahren nach einem der Ansprüche 1 bis 11,
wobei der Alkohol in Verfahrensschritt f) Methanol ist.

13. Verfahren nach einem der Ansprüche 1 bis 12,
wobei das Zuführen von CO₂ mit einem Druck erfolgt im Bereich von 1 MPa (10 bar) bis 5 MPa (50 bar).

14. Verfahren nach einem der Ansprüche 1 bis 13,
wobei das Zuführen von H₂ mit einem Druck erfolgt im Bereich von 1 MPa (10 bar) bis 5 MPa (50 bar).

15. Verfahren nach einem der Ansprüche 1 bis 14,
wobei das Erwärmen auf eine Temperatur erfolgt im Bereich von 80 °C bis 160 °C.
